# EUROPEAN PATENT APPLICATION

(11) **EP 4 810 151 A1**
(43) Date of publication of application: **23.09.2026**
(21) Application number: 24898075.7
(22) Date of filing: 26.11.2024
(51) Int. Cl.: B01J 19/00, C07K 1/04

(54) **COMPOSITE SOLID SUPPORT FOR BIOLOGICAL POLYMER SYNTHESIS**

(30) Priority: 30.11.2023 KR 20230170859; 13.11.2024 KR 20240160644
(71) Applicant: SP2TX Inc., Seoul 08826 (KR)
(72) Inventor: CHOI, Jae Sun, Seoul 08813 (KR)
(74) Representative: WSL Patentanwälte Partnerschaft mbB
(86) International application number: PCT/KR2024/018857
(87) International publication number: WO 2025/116481

(57) **Abstract**

The present disclosure relates to a composite solid support for synthesizing a biological polymer with high purity, high yield, and in large quantities, a method of preparing the same, and a method of synthesizing a biological polymer using the same.

## Description

### Technical Field

The present disclosure relates to a composite solid support for synthesizing a biological polymer with high purity, high yield, and in large quantities, a method of preparing the same, and a method of synthesizing a biological polymer using the same.

### Background Art

A process of synthesizing a biological polymer, such as a peptide or an oligonucleotide, consists of repetitive unit processes. The unit process consists of a coupling reaction and a deprotection reaction. The coupling reaction is a reaction in which a monomer, such as an amino acid or a nucleotide, is sequentially covalently bonded one at a time to synthesize a biological polymer of a target sequence. The deprotection reaction is a reaction in which a protecting group attached to the monomer is removed to prevent a side reaction during the coupling reaction and to prepare for a subsequent coupling reaction. In a biological polymer synthesis reaction, sequential addition of monomers is required, and a process of removing unreacted materials and by-products after the coupling reaction and the deprotection reaction performed for the sequential addition and separating only a desired product with high yield and high purity is important.

Methods for synthesizing a biological polymer include a liquid-phase synthesis method and a solid-phase synthesis method. In liquid-phase peptide synthesis (LPPS), a precipitation method, a crystallization method, or a chromatography method is used to separate a product of each of the coupling reaction and the deprotection reaction. These separation methods require considerable time and cost and have a limitation in that a loss rate in each unit process is 1.0 % or more. The loss rate in such a separation process causes a problem of sharply decreasing a synthesis yield as a sequence of the biological polymer becomes longer.

Solid-phase peptide synthesis (SPPS) was introduced to overcome these limitations, mainly based on the research of Bruce Merrifield (1984 Nobel Prize in Chemistry). The solid-phase synthesis method is characterized in that a biological polymer of a specific sequence is synthesized stepwise on a solid support, and unreacted materials and by-products are removed by a physical separation method at each step to obtain a product with high yield. A representative separation method is a filtration method using a filter having a filtration diameter that allows a reaction solution to pass through but does not allow a solid support to pass through. In general, when a separation method using a filter is used, a unit process loss rate of the solid support is 0.1 % or less.

The solid-phase synthesis method has become a standard for synthesizing long biological polymers with high yield, and various studies have been conducted to develop solid supports having various and improved physical properties. The development of solid supports has been largely divided, from a material perspective, into a single material and a composite material, and from a form perspective, into a particulate form and a non-particulate form. The single material consists of a single substance, exhibits uniform physicochemical properties, and has an advantage in that a manufacturing process and a molding process are simple.

Representative single-material solid supports include particulate polystyrene/divinylbenzene copolymer (hereinafter, PS/DVB), crosslinked polyethylene glycol polymer, poly-ε-lysine/sebacic acid copolymer, and controlled pore glass (hereinafter, CPG), and non-particulate amino-polyacrylamide resin fiber, cellulose, and hydroxylated polypropylene (hydroxylated PP).

The solid support most commonly used commercially in the field of solid-phase synthesis of biological polymers is PS/DVB, which has a network molecular structure in which linear polystyrene (hereinafter, PS) polymer chains are crosslinked by divinylbenzene (hereinafter, DVB) in a mass ratio of 1 % to 2 %. PS/DVB is widely used because it is inexpensive, is physicochemically stable, and has a wide range of loading density (also referred to as reaction site density), but has a limitation in that it is a hydrophobic support. Due to the nature of a hydrophobic support, PS/DVB exhibits extremely low volume swelling characteristics under conditions of a polar solvent such as water or alcohol, and aggregation is likely to occur during the synthesis of a hydrophobic biological polymer.

A composite-material solid support refers to a new material in which two or more different materials are physically or chemically combined to compensate for disadvantages and maximize advantages of each material. Representative composite-material solid supports include, as particulate types, a PEG grafted PS/DVB copolymer (hereinafter, PEG@PS/DVB) and a magnetic nanoparticle (hereinafter, MNP), and as non-particulate types, a hydroxypropyl acrylate coated polypropylene membrane (hereinafter, HPA-PP) and a peptide synthetic film (hereinafter, PSF).

The PEG@PS/DVB copolymer is a composite material prepared by grafting hydrophilic PEG onto a PS/DVB particulate polymer support having excellent physical strength and hydrophobic physical properties, and has advantages of being capable of effectively synthesizing hydrophobic peptides, which are difficult to synthesize using a conventional PS/DVB solid support, and of providing high volume swelling characteristics in various solvent environments and a wide range of loading density. However, the PEG@PS/DVB copolymer has a disadvantage in that a manufacturing cost is high due to the complexity of a grafting process.

MNP is generally a particulate composite-material support prepared by coating a magnetic material such as iron oxide with silica (SiO₂), amino dextran, or the like. The magnetic material serves as a composite substrate that imparts magnetism and provides a particulate form, and the silica or amino dextran serves as a functional substrate that provides reaction sites. Due to the nature of the functional substrate, the functional substrate has no or insignificant volume swelling characteristics and thus relies on surface reactions, resulting in an extremely low loading density.

Peptide pharmaceuticals generate revenues of billions of dollars annually in diabetes, obesity, and oncology, and are expanding their scope to the development of new drugs for new diseases such as cardiovascular diseases and neurodegenerative diseases. Currently, the disadvantage of peptides, namely a short in vivo half-life, has been addressed through methods such as introducing a non-natural amino acid or a fatty acid linker having an albumin-binding function. Various peptide pharmaceuticals having a dosing cycle of one week or more are already commercially available on the market, and numerous orally administered candidate substances are currently undergoing clinical trials by the U.S. Food and Drug Administration (hereinafter, FDA). The production demand for peptide pharmaceuticals is increasing by nearly 10 % annually, and thus there is an urgent need for a new synthesis platform to meet a corresponding improvement in productivity.

In addition, in order to increase the production capacity of synthesizing various biological polymers including peptides, various types of reactors have been studied. Representative reactors include a batch-type reactor and a flow-type reactor.

The traditional batch-type reactor is the oldest form of reactor and has an advantage in that reference literature and data can be readily obtained. However, as the capacity of the reactor increases for mass synthesis, a ratio of reactor surface area per unit volume sharply decreases, and thus the batch-type reactor has a structural limitation in that a temperature deviation between the reactor surface and the interior of the reactor increases. To minimize the temperature deviation problem, a peptide synthesis reaction using a large batch-type reactor of 100 liters (hereinafter, L) or more is generally performed under a roomtemperature condition, and accordingly, the large batch-type reactor has disadvantages in that a reaction process time is excessively long and yield and purity are lowered. In addition, a synthesis yield and purity also decrease when mixing in the reactor is non-uniform.

The flow-type reactor is a relatively recently developed form of reactor, and unlike the batch-type reactor, the flow-type reactor has advantages in that a ratio of surface area per unit volume is substantially high, so that a temperature deviation is minimal and mixing of a reaction solution is performed uniformly. In addition, the flow-type reactor has advantages such as occupying a smaller volume compared to the batch-type reactor, high ease of real-time reaction analysis through the introduction of an in-line detector, and ease of introducing process automation.

Representative methods of synthesizing and producing a biological polymer using a flow-type reactor include a packed particulate solid support column (hereinafter, PC) method and a volume-adjustable packed particulate solid support column (hereinafter, VA-PC) method. The above methods using the flow-type reactor for solid-phase synthesis of a biological polymer are known to perform reactions under high-temperature conditions of generally 50 °C or higher, thereby significantly shortening a required reaction time compared to the batch-type reactor and simultaneously achieving higher yield and purity compared to the batch-type reactor. However, it has been reported that in the case of using PC, backpressure increases to nearly 50 bar, or in the case of using VA-PC, a flow rate of the reactor must be used slowly to keep backpressure low.

When backpressure of the flow-type reactor is high or a flow rate is small, a temperature deviation in the reactor increases and non-uniform mixing occurs, and thus the advantages of the flow-type reactor are lost. In the flow-type reactor using PC or VA-PC, backpressure increases as an amount of the packed solid support increases or as a flow rate increases, and thus these methods can be regarded as unsuitable for mass production of a biological polymer using a large amount of solid support and as suitable for small-scale production using a small amount of solid support.

### Disclosure of Invention

### Technical Problem

Conventional solid supports have limitations in many aspects. First, since a solid support lacking a volume swelling characteristic in a solvent undergoes reactions only at a small number of externally exposed reaction sites, the solid support is suitable for diagnostic purposes such as screening rather than for production. CPG, MNP, HPA-PP, and PSF were respectively prepared in forms having a small particle size or coated with a membrane or film to expand the surface area and thereby increase the loading density, but nevertheless, these supports failed to reach a meaningful level of synthesis efficiency.

A particulate solid support has a small surface area per unit volume, which presents a limitation in terms of reaction rate. In a solid support having a volume swelling characteristic, a reactant present in a liquid phase diffuses from the outside of the solid support to the inside due to a concentration difference, and a reaction can proceed even at a reaction site inside the solid support. Accordingly, the solid support has a structural characteristic in which reactivity decreases as the distance from the surface of the solid support increases. Therefore, the solid support in a particulate form having a relatively small surface area per unit volume has structural limitations in terms of the diffusion of reactants and the reaction rate.

In addition, the particulate solid support has a structural limitation in that self-standing is impossible, and thus has structural characteristics unsuitable for a flow-type reactor. Due to the nature of being a particle, the particulate solid support has a characteristic of being moved by an external force such as gravity or a fluid flow. Therefore, a packing phenomenon caused by the flow of the reaction solution occurs inside the flow-type reactor, and an increase in friction and an elevation in backpressure occur due to a concomitant decrease in voids between the solid support particles. The increase in friction and the elevation in backpressure cause the generation of frictional heat inside the packed solid support and a decrease in flow rate, thereby reducing the uniformity of temperature and mixing, which are advantages of the flow-type reactor.

However, the problems to be solved by the present disclosure are not limited to the above-mentioned problems, and other unmentioned problems will be clearly understood by those skilled in the art from the following description.

### Solution to Problem

A first aspect of the present disclosure provides a composite solid support for synthesizing a biological polymer, the composite solid support including: a structure core having a length of 1 mm or more and a shape of at least one dimension; and a functional coating disposed on the structure core, wherein the biological polymer is synthesized in the functional coating.

A second aspect of the present disclosure provides a use of a composite solid support for synthesizing a biological polymer, wherein the composite solid support includes: a structure core having a length of 1 mm or more and a shape of at least one dimension; and a functional coating disposed on the structure core, wherein the biological polymer is synthesized in the functional coating.

A third aspect of the present disclosure provides a method of preparing a composite solid support for synthesizing a biological polymer, the method including: preparing a functional coating solution by mixing a main monomer, an active monomer, an initiator, and a solvent; impregnating a structure core having a length of 1 mm or more and a shape of at least one dimension with the functional coating solution; and polymerizing the functional coating solution applied to the structure core.

A fourth aspect of the present disclosure provides a method of synthesizing a biological polymer, the method including: coupling a first amino acid residue to which a protecting group is linked to the composite solid support according to the first aspect; removing the protecting group; and coupling a second amino acid residue to which a protecting group is linked to an N-terminus or a C-terminus of the previously coupled first amino acid from which the protecting group has been removed.

### Advantageous Effects of Invention

The composite solid support of the present disclosure has a form in which a functional coating having a volume swelling characteristic under various solvent conditions surrounds or is attached to a structure core, and features providing a large number of reaction sites where a biological polymer such as a peptide can be mass-synthesized with high purity and high yield.

Since the composite solid support of the present disclosure includes a structure core capable of self-standing, the composite solid support does not move according to the flow of a reaction solution inside a flow-type reactor, and unlike a case in which a particulate solid support incapable of self-standing is packed, the composite solid support features inducing a minimal increase in backpressure in the reactor. Due to this, the composite solid support of the present disclosure can be applied to a flow-type reactor as well as a batch-type reactor to be used for synthesizing a biological polymer.

Since a loading density and the purity and yield of biological polymer synthesis can be adjusted through the selection of a monomer in preparing the functional coating providing the reaction sites, the composite solid support of the present disclosure features that the composite solid support can be used for synthesizing various biological polymers such as peptides, oligonucleotides, and peptide nucleic acids (PNAs).

### Brief Description of Drawings

FIG. 1 is a graph comparing the expansion rate of a functional coating polymer according to an embodiment with the expansion rate of a conventional particulate solid-phase synthesis polymer (PS/DVB).
FIG. 2 shows photographs illustrating an appearance (a) of a structure core prior to coating used for preparing a composite solid support according to an embodiment and an appearance (b) of the composite solid support on which a functional coating is formed.
FIG. 3 shows a confocal laser scanning microscopy (CLSM) image (a) and an SEM image (b) illustrating a state in which a functional coating is formed in a composite solid support prepared using a structure core on which a hydrophilic coating (pretreatment) was performed according to an embodiment.
FIG. 4 shows confocal laser scanning microscopy (CLSM) images (a, b) and SEM images (c, d) illustrating a state in which a functional coating is formed in a composite solid support prepared using a structure core on which a hydrophilic coating (pretreatment) was not performed according to an embodiment.
FIG. 5A is an image showing an appearance and a morphological schematic diagram of a homogeneous composite solid support prepared according to an embodiment.
FIG. 5B is an image showing an appearance and a morphological schematic diagram of a heterogeneous composite solid support prepared according to an embodiment.
FIG. 5C is an image showing an appearance and a morphological schematic diagram of a particulate solid support used for conventional solid-phase synthesis.
FIG. 6 is a graph comparing coupling yields according to coupling reaction times of a conventional particulate solid support and a homogeneous/heterogeneous composite solid support according to an embodiment.
FIG. 7A is a graph confirming the purity and yield of an acyl carrier protein (hereinafter, ACP) (65-74), which is a model peptide synthesized in a batch-type reactor using a homogeneous composite solid support according to an embodiment.
FIG. 7B is a graph confirming the purity and yield of ACP (65-74) synthesized in a batch-type reactor using a conventional particulate solid support.
FIG. 8 is a graph confirming the purity and yield of ACP (65-74) synthesized in a flow-type reactor using a homogeneous composite solid support according to an embodiment.
FIG. 9A is a graph confirming the purity and yield of ACP (65-74) synthesized at a flow rate of 100 mL/min in a flow-type reactor using a composite solid support according to an embodiment.
FIG. 9B is a graph confirming the purity and yield of ACP (65-74) synthesized at a flow rate of 200 mL/min in a flow-type reactor using a composite solid support according to an embodiment.
FIG. 9C is a graph confirming the purity and yield of ACP (65-74) synthesized at a flow rate of 300 mL/min in a flow-type reactor using a composite solid support according to an embodiment.
FIG. 10 is a graph confirming the synthesis purity of ACP (65-74) according to a functional coating content (thickness and mass) of a composite solid support according to an embodiment.

### Mode for the Invention

Throughout the present specification, when any part is described as being "connected" to another part, this includes not only a case of being "directly connected" but also a case of being "electrically connected" with another element interposed therebetween.

Throughout the present specification, when any member is described as being positioned "on" another member, this includes not only a case where the member is in contact with the other member but also a case where another member exists between the two members.

Throughout the present specification, when any part is described as "including" any component, this means that the part may further include other components rather than excluding the other components unless specifically stated to the contrary. Terms of degree such as "about" and "substantially" used throughout the present specification are used in the sense of at or close to a numerical value when manufacturing and material tolerances inherent in the stated meaning are presented, and these terms are used to prevent an unscrupulous infringer from unfairly using the disclosure in which an exact or absolute numerical value is mentioned to help the understanding of the present disclosure.

The term "step of (doing)" or "step of" used throughout the present specification does not mean a "step for".

Throughout the present specification, the term "combination(s) thereof" included in a Markush-type expression means a mixture or combination of one or more selected from the group consisting of components described in the Markush-type expression, and means including one or more selected from the group consisting of the components.

Throughout the present specification, the description of "A and/or B" means "A or B, or A and B".

Throughout the present specification, the description of "loading density" means "the number of moles of functional groups provided per unit mass of a composite solid support," and may mean the density of synthesis reaction sites for a biological polymer.

Throughout the present specification, the description of a "biological polymer" means "a sequence-defined biopolymer, which is an arbitrary molecule including at least two chemically linked monomer units".

Hereinafter, embodiments and examples of the present disclosure will be described in detail with reference to the accompanying drawings. However, the present disclosure may not be limited to these embodiments, examples, and drawings.

A first aspect of the present disclosure provides a composite solid support for synthesizing a biological polymer, the composite solid support including: a structure core having a length of 1 mm or more and a shape of at least one dimension; and a functional coating disposed on the structure core, wherein the biological polymer is synthesized in the functional coating.

A second aspect of the present disclosure provides a use of a composite solid support for synthesizing a biological polymer, wherein the composite solid support includes: a structure core having a length of 1 mm or more and a shape of at least one dimension; and a functional coating disposed on the structure core, wherein the biological polymer is synthesized in the functional coating.

In an embodiment of the present disclosure, the functional coating may have a characteristic of swelling in a solvent.

In an embodiment of the present disclosure, the functional coating may include a functional group on a surface, in an interior, or both. Specifically, the functional coating includes the functional group, and due to the characteristic of swelling in a solvent, penetration of a reactant into the interior of the functional coating due to a concentration gradient readily occurs. Due to this, since the functional groups on the surface and in the interior of the functional coating are readily exposed to the reactant, the functional coating may exhibit high synthesis efficiency.

In an embodiment of the present disclosure, the functional group may include one or more selected from an amine group, a carboxyl group, a hydroxyl group, a carbonyl group, an amino group, a thiol group, and a phosphate group.

In an embodiment of the present disclosure, a loading density of the functional coating may be 0.01 mmol/g to 2 mmol/g. Specifically, a reaction site loading density of the functional coating may be 0.01 mmol/g to 2 mmol/g, 0.05 mmol/g to 2 mmol/g, 0.1 mmol/g to 2 mmol/g, 0.3 mmol/g to 2 mmol/g, 0.5 mmol/g to 2 mmol/g, 0.7 mmol/g to 2 mmol/g, 1 mmol/g to 2 mmol/g, 0.01 mmol/g to 1.7 mmol/g, 0.05 mmol/g to 1.7 mmol/g, 0.1 mmol/g to 1.7 mmol/g, 0.3 mmol/g to 1.7 mmol/g, 0.5 mmol/g to 1.7 mmol/g, 0.7 mmol/g to 1.7 mmol/g, 1.0 mmol/g to 1.7 mmol/g, 0.01 mmol/g to 1.5 mmol/g, 0.05 mmol/g to 1.5 mmol/g, 0.1 mmol/g to 1.5 mmol/g, 0.3 mmol/g to 1.5 mmol/g, 0.5 mmol/g to 1.5 mmol/g, 0.7 mmol/g to 1.5 mmol/g, 1.0 mmol/g to 1.5 mmol/g, 0.01 mmol/g to 1.2 mmol/g, 0.05 mmol/g to 1.2 mmol/g, 0.1 mmol/g to 1.2 mmol/g, 0.3 mmol/g to 1.2 mmol/g, 0.5 mmol/g to 1.2 mmol/g, 0.7 mmol/g to 1.2 mmol/g, 1.0 mmol/g to 1.2 mmol/g, 0.01 mmol/g to 1 mmol/g, 0.05 mmol/g to 1 mmol/g, 0.1 mmol/g to 1 mmol/g, 0.3 mmol/g to 1 mmol/g, 0.5 mmol/g to 1 mmol/g, or 0.7 mmol/g to 1 mmol/g, but is not limited thereto.

In an embodiment of the present disclosure, the functional coating may have an expansion amount per unit mass of 2 mL/g to 8 mL/g in water. Specifically, the functional coating may have an expansion amount per unit mass of 2 mL/g to 8 mL/g, 2 mL/g to 7 mL/g, 2 mL/g to 6 mL/g, 2 mL/g to 5 mL/g, 3 mL/g to 8 mL/g, 3 mL/g to 7 mL/g, 3 mL/g to 6 mL/g, or 3 mL/g to 5 mL/g in water, but is not limited thereto. In addition, since the functional coating undergoes swelling even in a solvent other than water, the functional coating may have a different expansion amount per unit mass depending on the type of solvent.

In an embodiment of the present disclosure, the functional coating may occupy a pore volume of 1 % to 15 % of the structure core without the functional coating. Specifically, the functional coating may occupy a pore volume of 1 % to 15 %, 1 % to 12 %, 1 % to 10 %, 1 % to 7 %, 1 % to 5 %, 1 % to 3 %, 3 % to 15 %, 3 % to 12 %, 3 % to 10 %, 3 % to 7 %, 3 % to 5 %, 5 % to 15 %, 5 % to 12 %, 5 % to 10 %, 5 % to 7 %, 7 % to 15 %, 7 % to 12 %, 7 % to 10 %, 10 % to 15 %, 10 % to 12 %, or 12 % to 15 % of the structure core without the functional coating, but is not limited thereto. In order to increase the efficiency of synthesizing a biological polymer, flowability of a reaction solution in the composite solid support is of critical importance. Therefore, it is necessary to appropriately adjust a coating amount and a thickness of the functional coating in the composite solid support so that the functional coating fills pores present in the structure core only to a desired level. In addition, since the functional coating has a swelling characteristic, the functional coating must be designed in consideration of this. If the functional coating fills less than 1 % of the pore volume of the structure core, synthesis of a desired amount of the biological polymer may not be achieved, and if the functional coating fills a volume exceeding 15 %, the reaction solution cannot flow smoothly, inevitably increasing a pressure drop.

In an embodiment of the present disclosure, a mass of the functional coating may be 5 % to 200 % relative to a mass of the structure core without the functional coating. Specifically, the mass of the functional coating may be 5 % to 200 %, 5 % to 150 %, 5 % to 120 %, 5 % to 100 %, 5 % to 80 %, 5 % to 50 %, 5 % to 30 %, 5 % to 10 %, 10 % to 200 %, 10 % to 150 %, 10 % to 120 %, 10 % to 100 %, 10 % to 80 %, 10 % to 50 %, 10 % to 30 %, 20 % to 200 %, 20 % to 150 %, 20 % to 120 %, 20 % to 100 %, 20 % to 80 %, 20 % to 50 %, 20 % to 30 %, 30 % to 200 %, 30 % to 150 %, 30 % to 120 %, 30 % to 100 %, 30 % to 80 %, 30 % to 50 %, 50 % to 200 %, 50 % to 150 %, 50 % to 120 %, 50 % to 100 %, 50 % to 80 %, 80 % to 200 %, 80 % to 150 %, 80 % to 120 %, 80 % to 100 %, 100 % to 200 %, 100 % to 150 %, or 100 % to 120 % relative to the mass of the structure core without the functional coating, but is not limited thereto. If the functional coating is coated in an amount of less than 5 % relative to the mass of the structure core, synthesis of a desired amount of the biological polymer may not be achieved, and if the functional coating is coated in an amount exceeding 200 %, the reaction solution cannot flow smoothly, inevitably increasing a pressure drop.

In an embodiment of the present disclosure, the structure core may have a characteristic of solvent resistance, thermal resistance, or both. Unlike the functional coating, the structure core lacks a characteristic of swelling and a characteristic of dissolving in a solvent, and thus the structure core may serve as a support for the composite solid support. In addition, since the structure core has thermal resistance, the structure core may remain unaltered and serve as a support even when synthesis of a biological polymer proceeds at a high temperature.

In an embodiment of the present disclosure, a component of the structure core may include one or more selected from a polymer, a metal, and a ceramic. Specifically, the polymer may include one or more selected from high-density polyethylene (HDPE), low-density polyethylene (LDPE), linear low-density polyethylene (LLDPE), ultra-high-molecular-weight polyethylene (UHMWPE), polypropylene (PP), polyethylene terephthalate (PET), polybutylene terephthalate (PBT), polyethylene naphthalate (PEN), polyamide (PA), polyvinyl chloride (PVC), polyvinylidene fluoride (PVDF), polyacetal, polycarbonate (PC), polyimide (PI), polyether ether ketone (PEEK), polyether sulfone (PES), polyphenylene oxide (PPO), and polyphenylene sulfide (PPS), but is not limited thereto. The metal may include one or more selected from stainless steel, titanium, nickel, tantalum, zirconium, and alloys thereof, but is not limited thereto. The ceramic may include one or more selected from fused silica, alumina, zirconia, silicon carbide, silicone nitride, boron nitride, and titanium diboride, but is not limited thereto.

In an embodiment of the present disclosure, the structure core may have one or more shapes selected from a one-dimensional shape, a two-dimensional shape, and a three-dimensional shape.

In an embodiment of the present disclosure, the one-dimensional shape may include one or more selected from a staple fiber, a filament fiber, and a rod. As a specific example, the one-dimensional shape of the structure core may include a fiber shape, and in this case, the structure core is configured with a length sufficient for a biological polymer synthesis to occur, and the composite solid support may be utilized in a biological polymer synthesis reaction by being packed in a reactor in an entangled form.

In an embodiment of the present disclosure, the two-dimensional shape may include one or more selected from a spunbond non-woven fabric, a meltblown non-woven fabric, a needle-punched non-woven fabric, a hydroentangled non-woven fabric, a woven fabric, a knitted fabric, a porous membrane, a polymeric film, and a mesh. As a specific example, the two-dimensional shape of the structure core may include a form in which a plurality of the structure cores having the one-dimensional shape are randomly arranged. In addition, the random arrangement forms pores between the plurality of structure cores having the one-dimensional shape, and thus the two-dimensional shape may exhibit porosity.

In an embodiment of the present disclosure, the three-dimensional shape may include an open-cell foam, a macro-pored sphere, or both.

In an embodiment of the present disclosure, the structure core may have a cross-sectional length of 1 mm or more, and the structure core may have a size sufficient for the functional coating to participate in synthesizing a desired biological polymer. As a specific example, when the structure core has a one-dimensional shape, the structure core having a shape such as a fiber may be implemented to serve as a support for the composite solid support through entanglement. In addition, when the structure core has a two-dimensional or three-dimensional shape, the structure core itself may serve as a support, such as a non-woven fabric (two-dimensional) and a foam (three-dimensional). Therefore, the cross-sectional length of the structure core may be 1 mm or more, and a one-dimensional structure core having a theoretically infinite length may also serve as a support.

In an embodiment of the present disclosure, the functional coating may include a polymer of one or more main monomers and an active monomer. As a specific example, the functional coating may include a polymer of a first monomer and the active monomer, or the functional coating may include a polymer of a first monomer, a second monomer, and the active monomer. In addition, the functional coating may be a polymer formed through a polymerization reaction of one or more main monomers serving as main components with the active monomer providing a reaction site.

In an embodiment of the present disclosure, the composite solid support may include a homogeneous composite solid support, a heterogeneous composite solid support, or both. Specifically, in the homogeneous composite solid support, the functional coating may be formed of the polymer of the one or more main monomers and the active monomer. In addition, in the heterogeneous composite solid support, the functional coating including a pulverized conventional solid support may be coated on the structure core. The conventional solid support may include one or more selected from a polystyrene/divinylbenzene copolymer (PS/DVB), a crosslinked polyethylene glycol polymer, a poly-ε-lysine/sebacic acid copolymer, a controlled pore glass, an amino-polyacrylamide resin fiber, cellulose, and a hydroxylated polypropylene, but is not limited thereto.

In an embodiment of the present disclosure, the one-dimensional structure core may have a diameter of 10 µm to 100 µm. Specifically, the diameter may be 10 µm to 100 µm, 20 µm to 100 µm, 30 µm to 100 µm, 40 µm to 100 µm, 10 µm to 80 µm, 20 µm to 80 µm, 30 µm to 80 µm, 40 µm to 80 µm, 10 µm to 60 µm, 20 µm to 60 µm, 30 µm to 60 µm, 40 µm to 60 µm, 10 µm to 50 µm, 20 µm to 50 µm, 30 µm to 50 µm, or 40 µm to 50 µm, but is not limited thereto. If the diameter of the one-dimensional structure core is less than 10 µm, the structure core becomes too thin, causing a problem in which the structure core aggregates and mechanical properties are degraded, and if the diameter exceeds 100 µm, an amount of the coated functional coating becomes excessively small, which may cause a problem in which an amount of the synthesized biological polymer decreases.

In an embodiment of the present disclosure, the two-dimensional structure core may have a thickness of 10 µm to 10 mm. Specifically, the two-dimensional structure core may have a thickness of 10 µm to 10 mm, 50 µm to 10 mm, 100 µm to 10 mm, 150 µm to 10 mm, 200 µm to 10 mm, 250 µm to 10 mm, 300 µm to 10 mm, 350 µm to 10 mm, 400 µm to 10 mm, 10 µm to 5 mm, 50 µm to 5 mm, 100 µm to 5 mm, 150 µm to 5 mm, 200 µm to 5 mm, 250 µm to 5 mm, 300 µm to 5 mm, 350 µm to 5 mm, 400 µm to 5 mm, 10 µm to 3 mm, 50 µm to 3 mm, 100 µm to 3 mm, 150 µm to 3 mm, 200 µm to 3 mm, 250 µm to 3 mm, 300 µm to 3 mm, 350 µm to 3 mm, 400 µm to 3 mm, 10 µm to 1 mm, 50 µm to 1 mm, 100 µm to 1 mm, 150 µm to 1 mm, 200 µm to 1 mm, 250 µm to 1 mm, 300 µm to 1 mm, 350 µm to 1 mm, 400 µm to 1 mm, 10 µm to 0.5 mm, 50 µm to 0.5 mm, 100 µm to 0.5 mm, 150 µm to 0.5 mm, 200 µm to 0.5 mm, 250 µm to 0.5 mm, 300 µm to 0.5 mm, 350 µm to 0.5 mm, or 400 µm to 0.5 mm, but is not limited thereto. If the thickness of the two-dimensional structure core is less than 10 µm, it is difficult for the structure core to maintain mechanical properties, and if the thickness exceeds 10 mm, there is a possibility that a polymerization reaction may not occur uniformly up to the inside of the functional coating.

In an embodiment of the present disclosure, a porosity of the structure core may be 50 % to 95 %. When the structure core is one-dimensional, the structure core having a shape such as a fiber may form pores through entanglement, and when the structure core is two-dimensional or three-dimensional, the structure core may include pores therein, such as a non-woven fabric and a foam. In the case of having pores as such, the porosity of the structure core may be 50 % to 95 %, 60 % to 95 %, 70 % to 95 %, 80 % to 95 %, 90 % to 95 %, 50 % to 90 %, 60 % to 90 %, 70 % to 90 %, or 80 % to 90 %, but is not limited thereto. Here, a pore size of the structure core is not particularly limited, and if the porosity is less than 50 %, the pores become blocked when the functional coating swells, and thus synthesis of a biological polymer may not occur up to the inside of the functional coating. In addition, if the porosity exceeds 95 %, it may be difficult for the structure core to maintain mechanical properties.

In an embodiment of the present disclosure, a thickness of the functional coating may be 0.1 µm to 1000 µm. The thickness of the functional coating may be selected to be an appropriate thickness depending on a type of a desired biological polymer and whether the structure core has a one-dimensional, two-dimensional, or three-dimensional shape. Specifically, the thickness of the functional coating may be 0.1 µm to 1000 µm, 0.5 µm to 1000 µm, 1 µm to 1000 µm, 5 µm to 1000 µm, 10 µm to 1000 µm, 50 µm to 1000 µm, 100 µm to 1000 µm, 0.1 µm to 500 µm, 0.5 µm to 500 µm, 1 µm to 500 µm, 5 µm to 500 µm, 10 µm to 500 µm, 50 µm to 500 µm, 100 µm to 500 µm, 0.1 µm to 200 µm, 0.5 µm to 200 µm, 1 µm to 200 µm, 5 µm to 200 µm, 10 µm to 200 µm, 50 µm to 200 µm, 100 µm to 200 µm, 0.1 µm to 100 µm, 0.5 µm to 100 µm, 1 µm to 100 µm, 5 µm to 100 µm, 10 µm to 100 µm, 50 µm to 100 µm, 0.1 µm to 50 µm, 0.5 µm to 50 µm, 1 µm to 50 µm, 5 µm to 50 µm, 10 µm to 50 µm, 0.1 µm to 20 µm, 0.5 µm to 20 µm, 1 µm to 20 µm, 5 µm to 20 µm, or 10 µm to 20 µm, but is not limited thereto. If the thickness of the functional coating is excessively small, such as less than 0.1 µm, an amount of the functional coating is extremely small, which causes a problem in which an amount of the synthesized biological polymer becomes extremely small. In addition, if the thickness of the functional coating is excessively large, such as exceeding 1000 µm, the functional coating itself is prepared by a non-uniform polymerization reaction, which may cause a problem in which purity decreases during a synthesis process of the biological polymer.

A third aspect of the present disclosure provides a method of preparing a composite solid support for synthesizing a biological polymer, the method including: preparing a functional coating solution by mixing one or more main monomers, an active monomer, an initiator, and a solvent; impregnating a structure core having a length of 1 mm or more and a shape of at least one dimension with the functional coating solution; and polymerizing the functional coating solution applied to the structure core.

In an embodiment of the present disclosure, the structure core impregnated with the functional coating solution may have a surface that is hydrophilically treated or untreated. Specifically, the surface hydrophilic treatment may be performed through a method selected from a chemical method using a surfactant or an acidic solution, and a physical method including a plasma treatment or UV irradiation, but is not limited thereto.

In an embodiment of the present disclosure, a loading density may be adjusted according to a length of the main monomer.

In an embodiment of the present disclosure, the main monomer may include a first monomer, or may include a first monomer and a second monomer.

In an embodiment of the present disclosure, the functional coating solution may be prepared by mixing a first monomer, an active monomer, an initiator, and a solvent. In this case, the first monomer serves as a crosslinking agent, and by adjusting a length thereof, a loading density provided through the added active monomer can be adjusted. If the length of the first monomer is short, the loading density may increase, but steric hindrance may occur between synthesized biological polymers. Therefore, an appropriate selection of the first monomer is required depending on a target biological polymer.

In an embodiment of the present disclosure, the first monomer may include one or more selected from a bisacrylamide-based crosslinking agent, a triazine substituted with a methacryloyl group or an alkenyl group, and an acrylate-based crosslinking agent, and specifically, the first monomer may include a polyethylene glycol-based material, for example, polyethylene glycol diacrylate. Specifically, any material may be used as the first monomer without limitation as long as the material is capable of reacting or bonding with the active monomer to expose a functional group to an interior and/or a surface in the functional coating. As a non-limiting example, the first monomer may include one or more selected from polyethylene glycol diacrylate, N,N'-methylenebisacrylamide (MBA), ethylene glycol dimethacrylate (EGDMA), poly(ethylene glycol) dimethacrylate (PEGDMA), glycidyl methacrylate (GMA), divinyl sulfone (DVS), triethylene glycol divinyl ether (TEGDVE), and diallyl phthalate (DAP).

In an embodiment of the present disclosure, the active monomer is for providing a functional group to an interior and/or a surface of the functional coating, and the active monomer may include one or more selected from an acrylate-based material, an acrylamide-based material, and a methacrylamide-based material. As a non-limiting example, the active monomer may include one or more selected from N-(2-aminopropyl)methacrylamide hydrochloride, aminoethyl methacrylate hydrochloride (AEMA·HCl), 2-aminoethyl methacrylate (2-AEMA), N-(3-aminopropyl)methacrylamide hydrochloride (APMA), 4-aminostyrene, N-(4-aminophenyl)methacrylamide, N,N-dimethylaminoethyl methacrylate (DMAEMA), N,N-dimethylaminopropylacrylamide (DMAPAA), and N-(2-aminoethyl)acrylamide hydrochloride.

In an embodiment of the present disclosure, the initiator may include a photoinitiator, a thermal initiator, or both. The photoinitiator may be a UV initiator, and may include one or more selected from 1-hydroxycyclohexyl phenyl ketone (Irgacure 907), 2-methyl-1-[4-(methylthio)phenyl]-2-morpholinopropan-1-one (Irgacure 184C), 1-hydroxy-2-methyl-1-phenylpropan-1-one (Darocur 1173), a mixed initiator of Irgacure 184C and benzophenone (Irgacure 500), a mixed initiator of Irgacure 184C and Irgacure 1173 (Irgacure 1000), 2-hydroxy-1-[4-(2-hydroxyethoxy)phenyl]-2-methyl-1-propanone (Irgacure 2959), methyl benzoylformate (Darocur MBF), α,α-dimethoxy-α-phenylacetophenone (Irgacure 651), 2-benzyl-2-(dimethylamino)-1-[4-(4-morpholinyl)phenyl]-1-butanone (Irgacure 369), a mixed initiator of Irgacure 369 and Irgacure 651 (Irgacure 1300), diphenyl(2,4,6-trimethylbenzoyl)phosphine oxide (Darocur TPO), a mixed initiator of Darocur TPO and Darocur 1173 (Darocur 4265), phenylbis(2,4,6-trimethylbenzoyl)phosphine oxide (Irgacure 819), a mixed initiator of Irgacure 819 and Darocur 1173 (Irgacure 2005), a mixed initiator of Irgacure 819 and Darocur 1173 (Irgacure 2010), a mixed initiator of Irgacure 819 and Darocur 1173 (Irgacure 2020), bis(η⁵-2,4-cyclopentadien-1-yl)bis[2,6-difluoro-3-(1H-pyrrol-1-)phenyl]titanium (Irgacure 784), and a mixed initiator containing 2-hydroxy-2-methylpropiophenone and benzophenone (HSP 188).

In addition, the thermal initiator may include one or more selected from azo-based compounds such as 2,2'-azobis(2-methylbutyronitrile), 2,2'-azobisisobutyronitrile, 2,2'-azobis(2,4-dimethylvaleronitrile), 4,4'-azobis(4-cyanovaleric acid), 1,1'-azobis(cyclohexanecarbonitrile), and 2,2'-azobis(4-methoxy-2,4-dimethylvaleronitrile); peroxy-based compounds such as tetramethylbutyl peroxyneodecanoate (e.g., Perocta ND, manufactured by NOF Corp.), bis(4-butylcyclohexyl) peroxydicarbonate (e.g., Peroyl TCP, manufactured by NOF Corp.), di(2-ethylhexyl) peroxycarbonate, butyl peroxyneodecanoate (e.g., Perbutyl ND, manufactured by NOF Corp.), dipropyl peroxydicarbonate (e.g., Peroyl NPP, manufactured by NOF Corp.), diisopropyl peroxydicarbonate (e.g., Peroyl IPP, manufactured by NOF Corp.), diethoxyethyl peroxydicarbonate (e.g., Peroyl EEP, manufactured by NOF Corp.), diethoxyhexyl peroxydicarbonate (e.g., Peroyl OEP, manufactured by NOF Corp.), hexyl peroxydicarbonate (e.g., Perhexyl ND, manufactured by NOF Corp.), dimethoxybutyl peroxydicarbonate (e.g., Peroyl MBP, manufactured by NOF Corp.), bis(3-methoxy-3-methoxybutyl) peroxydicarbonate (e.g., Peroyl SOP, manufactured by NOF Corp.), dibutyl peroxydicarbonate, dicetyl peroxydicarbonate, dimyristyl peroxydicarbonate, 1,1,3,3-tetramethylbutyl peroxypivalate, hexyl peroxypivalate (e.g., Perhexyl PV, manufactured by NOF Corp.), butyl peroxypivalate (e.g., Perbutyl, manufactured by NOF Corp.), trimethylhexanoyl peroxide (e.g., Peroyl 355, manufactured by NOF Corp.), dimethylhydroxybutyl peroxyneodecanoate (e.g., Luperox 610M75, manufactured by Atofina), amyl peroxyneodecanoate (e.g., Luperox 546M75, manufactured by Atofina), butyl peroxyneodecanoate (e.g., Luperox 10M75, manufactured by Atofina), t-butyl peroxyneoheptanoate, amyl peroxypivalate (e.g., Luperox 546M75, manufactured by Alofina), t-butyl peroxypivalate, t-amyl peroxy-2-ethylhexanoate, lauryl peroxide, dilauroyl peroxide, didecanoyl peroxide, benzoyl peroxide, dibenzoyl peroxide, 2,2-bis(tert-butylperoxy)butane, 1,1-bis(tert-butylperoxy)cyclohexane, 2,5-bis(butylperoxy)-2,5-dimethylhexane, 2,5-bis(tert-butylperoxy)-1-methylethyl)benzene, 1,1-bis(tert-butylperoxy)-3,3,5-trimethylcyclohexane, tert-butyl hydroperoxide, tert-butyl peroxide, tert-butyl peroxybenzoate, tert-butyl peroxyisopropyl carbonate, cumene hydroxyperoxide, dicumyl peroxide, lauroyl peroxide, and 2,4-pentanedione peroxide; and one or more selected from tert-butyl peracetate, peracetic acid, and potassium persulfate.

In an embodiment of the present disclosure, the solvent may include one or more selected from water (H₂O), ethanol (C₂H₅OH), methanol (CH₃OH), isopropanol (C₃H₈O), acetone (C₃H₆O), ethylene glycol (C₂H₆O₂), propylene glycol (C₃H₈O₂), acetonitrile (C₂H₃N), dimethylformamide (DMF, C₃H₇NO), dimethyl sulfoxide (DMSO, C₂H₆OS), N,N-dimethylacetamide (DMA, C₄H₉NO), tetrahydrofuran (THF, C₄H₈O), dichloromethane (DCM, CH₂Cl₂), and glycerin (C₃H₈O₃).

In an embodiment of the present disclosure, the functional coating solution may be prepared by mixing a first monomer, a second monomer, an active monomer, an initiator, and a solvent. The first monomer serves as a crosslinking agent, and the second monomer serves as a binder, and any material may be used as the second monomer without limitation as long as the material is capable of reacting or bonding with the first monomer and the active monomer. More specifically, the second monomer may include one or more selected from 2-hydroxyethyl acrylate, 2-hydroxyethyl methacrylate (HEMA), acrylic acid (AA), methyl methacrylate (MMA), ethyl acrylate (EA), butyl acrylate (BA), glycidyl methacrylate (GMA), and vinyl acetate (VAc).

In an embodiment of the present disclosure, the functional coating solution may further include a surfactant. Specifically, the surfactant may include one or more selected from sodium dodecyl sulfate (SDS), sodium lauryl ether sulfate (SLES), lauramine oxide, sodium myreth sulfate, cetyltrimethylammonium bromide (CTAB), Triton X-100, Tween 20, Tween 80, and decyl glucoside.

In an embodiment of the present disclosure, the functional coating solution may include 1 to 25 parts by weight of the active monomer with respect to 100 parts by weight of the main monomer. Specifically, the functional coating solution may include 1 to 25 parts by weight of the active monomer and 0.1 to 10 parts by weight of the initiator with respect to 100 parts by weight of the main monomer. More specifically, when the main monomer includes two types, the main monomer may be classified into a first monomer and a second monomer. In this case, the functional coating solution may include 0 to 50 parts by weight of the second monomer and 1 to 25 parts by weight of the active monomer with respect to 100 parts by weight of the first monomer, or may include 0 to 50 parts by weight of the second monomer, 1 to 25 parts by weight of the active monomer, 0.1 to 10 parts by weight of the initiator, and 0 to 25 parts by weight of the surfactant with respect to 100 parts by weight of the first monomer. If a ratio of the active monomer for exposing a functional group is high, an amount of the synthesized biological polymer may increase, but there is a high possibility of occurrence of steric hindrance between the biological polymers synthesized in the composite solid support. In addition, if a ratio of the first monomer (the main monomer) is high, durability of the functional coating may increase, but the amount of the synthesized biological polymer may decrease.

In an embodiment of the present disclosure, the functional coating solution may include 10 to 40 parts by weight of the main monomer, 1 to 10 parts by weight of the active monomer, 0.05 to 5 parts by weight of the initiator, and 30 to 80 parts by weight of the solvent with respect to 100 parts by weight of the composite solid support. Specifically, when the main monomer is a first monomer, the functional coating solution may include 10 to 40 parts by weight of the first monomer, 1 to 10 parts by weight of the active monomer, 0.05 to 5 parts by weight of the initiator, and 30 to 80 parts by weight of the solvent with respect to 100 parts by weight of the composite solid support. In the functional coating solution, the second monomer and the surfactant are not essential components, and these components may be added depending on synthesis of a target biological polymer compound. In this case, the functional coating solution may include 10 to 40 parts by weight of the first monomer, 0 to 10 parts by weight of the second monomer, 1 to 10 parts by weight of the active monomer, 0.05 to 5 parts by weight of the initiator, 0 to 10 parts by weight of the surfactant, and 30 to 80 parts by weight of the solvent with respect to 100 parts by weight of the composite solid support.

In an embodiment of the present disclosure, the biological polymer may include one or more selected from a peptide, an oligonucleotide, and a peptide nucleic acid (PNA), but is not limited thereto.

A fourth aspect of the present disclosure provides a method of synthesizing a biological polymer, the method including: coupling a first amino acid residue to which a protecting group is linked to the composite solid support according to the first aspect; removing the protecting group; and coupling a second amino acid residue to which a protecting group is linked to an N-terminus or a C-terminus of the previously coupled first amino acid from which the protecting group has been removed.

The method of synthesizing the biological polymer provides a process in which a target biological polymer can be synthesized in the functional coating by repeatedly performing the coupling, the removal of the protecting group, and the additional coupling while changing the amino acid.

In an embodiment of the present disclosure, the method of synthesizing the biological polymer may further include introducing a linker to the functional coating of the composite solid support before coupling the first amino acid residue to which the protecting group is linked.

In an embodiment of the present disclosure, the method of synthesizing the biological polymer may further include removing a protecting group from the introduced linker before coupling the first amino acid residue to which the protecting group is linked.

In an embodiment of the present disclosure, the linker may include one or more selected from a Rink amide linker, a Wang linker, a 2-chlorotrityl (CTC) linker, a Sieber linker, a BAL linker, a 4-sulfamylbutyryl linker, an HMBA (TFA-stable) linker, an HMPA [4-(hydroxymethyl)phenoxyacetylamidoethyl] linker, and an HMBA [4-(hydroxymethyl)benzoylamidoethyl] linker.

In an embodiment of the present disclosure, the functional coating has a functional group exposed on an interior and/or a surface thereof, and a linker may be bonded to the functional group. A biological polymer including a plurality of monomer units may be synthesized by adding an additional monomer unit (amino acid residue) to a monomer unit (amino acid residue) linked through the functional coating (functional group)-linker.

In an embodiment of the present disclosure, a type of the amino acid residue is not limited, and preferably, the amino acid residue may be selected from alanine, cysteine, aspartic acid, glutamic acid, phenylalanine, glycine, histidine, isoleucine, lysine, leucine, methionine, asparagine, pyrrolysine, proline, glutamine, arginine, serine, threonine, selenocysteine, valine, tryptophan, and tyrosine, but is not limited thereto.

### [Examples]

### Example 1. Preparation of Polymer for Functional Coating and Verification of Swelling Properties

### Example 1-1: Preparation of Polymer for Functional Coating

To prepare the polymer for the functional coating, each reagent shown in Table 1 below was used, and the reagents were mixed and stirred in a solvent to be uniformly dissolved. Here, the components of the polymer for the functional coating only need to satisfy the respective mass ratio (parts by weight) ranges, and any solvent may be used regardless of its type, as the solvent will be subsequently dried. In the experiment, the components were weighed such that the sum of each reagent and the solvent (D.W) was 100 g [the solvent may be used in an amount of 10 wt% to 90 wt% of the total (reagents + solvent)].

**[Table 1]**

| Category | Reagent Name | Parts by mass |
|---|---|---|
| First monomer | Polyethylene glycol diacrylate | 100 |
| Second monomer | Methyl acrylate | 0-50 |
| Active monomer | N-(2-aminopropyl) methacrylamide hydrochloride | 1-25 |
| Surfactant | Sodium dodecyl sulfate | 0-25 |

Thereafter, 0.5 g of 2-hydroxy-2-methylpropiophenone as a curing reaction initiator was added to the solution, and the mixture was uniformly mixed to prepare a functional coating solution. The functional coating solution was uniformly applied to a Petri dish, and an ultraviolet energy of 10 mJ/cm² to 1,000 mJ/cm² was sufficiently irradiated to cure the solution. After the curing was completed, reaction residues were removed using ethanol to obtain a functional coating polymer disk. The mass of the completely dried polymer for the functional coating was measured to be 45.7 g.

The prepared polymer for the functional coating was flash-frozen using liquid nitrogen and then crushed using a mortar. Thereafter, 100-mesh and 200-mesh sieves were used to obtain a particulate polymer for the functional coating having a particle size range of 100 to 200 mesh (74 to 149 µm) (Example 1).

### Example 1-2: Verification of Swelling Properties of Polymer for Functional Coating by Solvent

A tip of a 3 mL syringe was tightly blocked using glass fiber, and the weight of the syringe was measured. Thereafter, about 200 mg of the polymer for the functional coating was filled into the syringe, and an initial volume was measured (Initial Volume V1). Thereafter, 3 mL of each solvent for verifying swelling properties was added to the syringe filled with the polymer for the functional coating, and the syringe was left to stand for 30 minutes. Thereafter, a plunger was slowly pressed to remove the solvent, and the increased volume was measured (Final Volume V2).

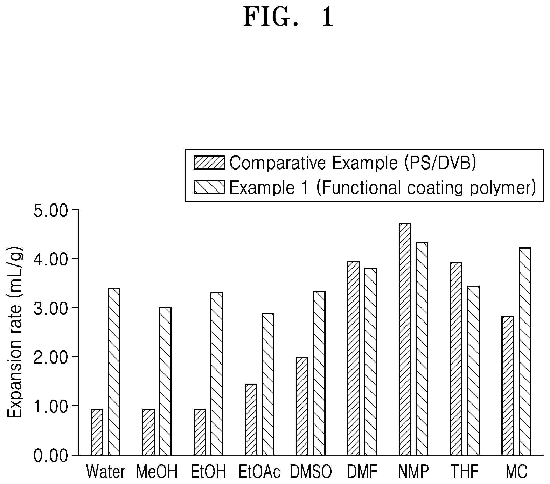

A volume change of the polymer for the functional coating caused by the solvent was calculated (V2 - V1), and an expansion amount per unit mass was calculated by dividing the volume change value by the mass of the polymer for the functional coating. Then, as a comparative example, an expansion amount per unit mass was calculated in the same manner using commercially available PS/DVB polymer (crosslinked polystyrene/divinyl benzene copolymer) particles (BeadTech, Rink amide AM(MBHA) resin, 0.5 mmol/g, 100 to 200 mesh) (Table 2 and FIG. 1).

**[Table 2]**

| Polymer | Expansion amount per unit mass by solvent (mL/g) | | | | | | | | |
|---|---|---|---|---|---|---|---|---|---|
| | H₂O | MeOH | EtOH | EtOAc | DMSO | DMF | NMP | THF | MC |
| Comparative Example | 0.95 | 0.96 | 1.00 | 1.44 | 1.99 | 3.96 | 4.72 | 3.94 | 2.84 |
| Example 1 | 3.39 | 3.00 | 3.33 | 2.87 | 3.34 | 3.81 | 4.34 | 3.44 | 4.23 |

Referring to the results of Table 2 and FIG. 1, it was confirmed that Example 1 exhibited a greater degree of expansion in solvents such as H₂O, EtOH, MeOH, EtOAc, DMSO, and MC compared to the comparative example. In particular, an expansion of 2 times or more was observed in eco-friendly solvents such as H₂O, EtOH, MeOH, and EtOAc. Through this, it was confirmed that Example 1 has superior swelling properties under eco-friendly solvent conditions compared to the conventional PS/DVB polymer.

### Example 2. Measurement of Loading Density of Functional Coating Polymer

### Example 2-1: Introduction of Fmoc-Gly-OH to Functional Coating Polymer

8 mL of DMF was added to 1 g of the functional coating polymer of Example 1, and the polymer was allowed to swell for 30 minutes, after which the DMF was filtered to prepare a sample for measuring the loading density. Then, 3 mmol of Fmoc(fluorenyl methoxycarbonyl)-Gly(glycine)-OH, DIC, and Oxyma pure (ethyl cyanohydroxyiminoacetate) were added to and dissolved in 8 mL of clean DMF to prepare a reagent. The reagent was added to the swollen functional coating polymer sample, and the mixture was stirred and reacted at room temperature for 5 hours. Thereafter, the reaction solution was discarded, and the sample was washed seven times with 8 mL of DMF, washed three times with 8 mL of ethanol, and dried under reduced pressure for 12 hours.

### Example 2-2: Measurement of Loading Density

8 mL of DMF was added to 1 g of the functional coating polymer into which Fmoc-Gly-OH had been introduced, and the polymer was allowed to swell for 30 minutes; then, 2 mL of a 20 % piperidine solution was added to the sufficiently swollen functional coating polymer, and the mixture was reacted at room temperature for 45 minutes. A 1 mL aliquot of the supernatant of the sample was collected, and the aliquot was diluted until the absorbance at 301 nm reached approximately 1, and the loading density was analyzed. As a result of calculating using Equation 1 below, the loading density was measured to be 0.17 mmole/g.$L = \left({A}_{301}\times V\times d\right) / \left(Ec\times W\times m\times 1000\right)$
Here, L = loading density (mmole/g)
A₃₀₁ = absorbance at 301 nm
V = volume of the deprotection solution (piperidine solution)
d = dilution factor
Ec = extinction coefficient
W = width of the cuvette
m = mass of the sample

### Example 3. Preparation of Composite Solid Support

To prepare a composite solid support, the functional coating solution (prior to curing) from 1-1) of Example 1 and 20 g of a structure core (polypropylene, spunbond non-woven fabric, 40 g/m²) were prepared. The structure core was sufficiently impregnated with the functional coating solution, and after the structure core was removed from the functional coating solution, ultraviolet energy of 10 mJ/cm² to 1,000 mJ/cm² was sufficiently irradiated onto the structure core in an ultraviolet curing chamber.

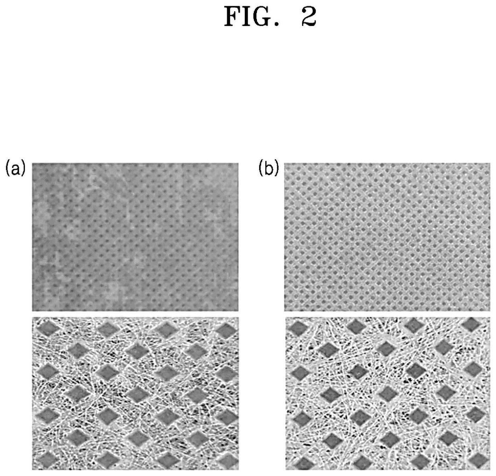

After the curing process was completed, the coated structure core was washed with ethanol to remove reaction residues, and the coated structure core was dried. A composite solid support in which the structure core was coated with the functional coating polymer was obtained (solid support 30.4 g, 60.8 g/m²); the appearance of the structure core prior to the coating process is as shown in FIG. 2A, and the appearance of the composite solid support is as shown in FIG. 2B.

### Example 4. Surface Treatment of Structure Core

Prior to impregnating the structure core in the functional coating polymer curing reaction solution in Example 3, a hydrophilic treatment was performed on the surface of the structure core. The surface hydrophilic treatment was performed by impregnating the structure core in a 2 % aqueous sodium dodecyl sulfate (SDS) solution for 1 minute, removing the structure core to eliminate excess aqueous SDS solution, and drying the structure core at room temperature for 12 hours. Thereafter, a composite solid support was prepared in the same manner as in Example 3, and the difference between this composite solid support and a composite solid support using a structure core not subjected to the surface hydrophilic treatment was evaluated. First, a fluorescein 5(6)-isothiocyanate (FITC) fluorescent substance was chemically conjugated to each composite solid support, and the fluorescent-tagged composite solid supports were analyzed by confocal laser scanning microscopy (CLSM). In addition, scanning electron microscope (SEM) images were obtained to precisely observe the coated states.

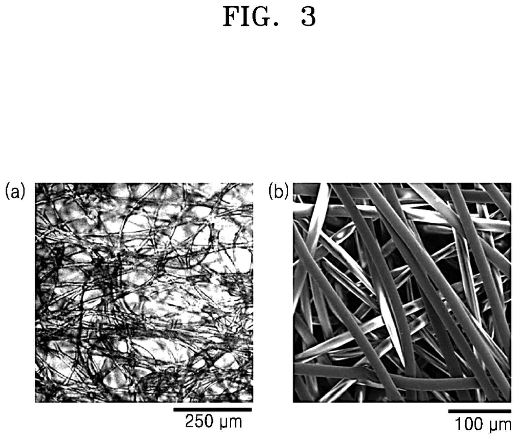

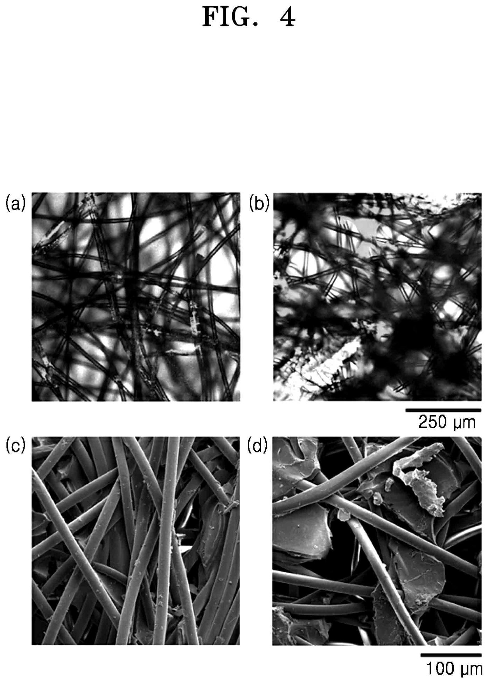

As shown in FIGS. 3A and 3B, the composite solid support using the structure core subjected to the surface hydrophilic treatment showed that the FITC fluorescent substance was evenly distributed along the structure core without aggregating, confirming that the functional coating was uniformly formed over the entire structure core. On the other hand, the composite solid support using the structure core not subjected to the surface hydrophilic treatment was confirmed to have a functional coating formed in an aggregated shape in various locations, as shown in FIGS. 4A, 4B, 4C, and 4D. This is a result of the surface of the structure core used in the experiment being hydrophobic while the functional coating polymer is hydrophilic, which implies that a more uniform functional coating can be formed if the surface treatment is appropriately performed depending on the types of the structure core and the functional coating polymer.

### Example 5. Preparation of Heterogeneous Composite Solid Support

Instead of the functional coating polymer of Example 1, a heterogeneous composite solid support in which a conventional PS/DVB polymer was coated on a structure core was prepared (hereinafter, the composite solid support prepared by curing the functional coating polymer of Example 3 is referred to as a homogeneous composite solid support, and the composite solid support coated with the conventional polymer is referred to as a heterogeneous composite solid support). The materials and methods of Example 3 were used, except that an active monomer was not used, and a conventional bead-type PS/DVB polymer for solid-phase synthesis pulverized to a size of 0.1 µm to 10 µm was used.

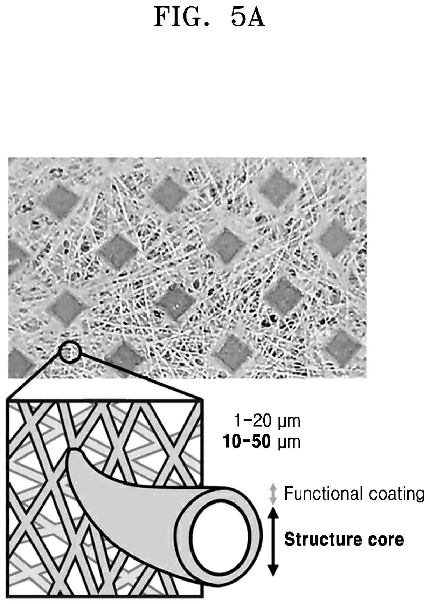

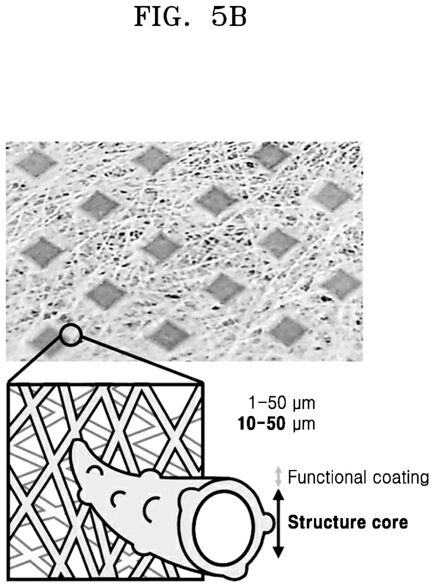

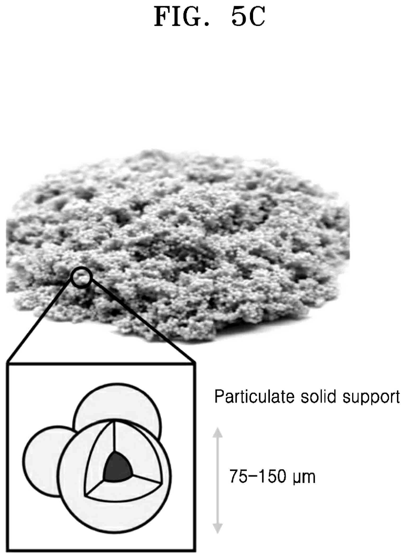

Referring to FIG. 5A, it can be confirmed that the composite solid support according to Example 3 is homogeneous, having the morphology of a composite solid support with a uniform surface. FIG. 5B shows a conventional bead-type PS/DVB polymer for solid-phase synthesis, and it can be confirmed that when this polymer is coated on the structure core, a heterogeneous composite solid support in which the structure core is ruggedly coated with a heterogeneous surface is prepared, as shown in FIG. 5C.

### Example 6. Coupling Kinetic Test Using Particulate Solid Support and Homogeneous/Heterogeneous Composite Solid Supports

The homogeneous composite solid support subjected to the surface treatment of Example 4, the heterogeneous composite solid support of Example 5, and the particulate solid support (PS/DVB polymer) were each placed into a reactor, and a coupling kinetic test was performed through the reaction steps in Table 3 below. First, 5 mL of DMF was added to each reactor, and the solid supports were sufficiently swollen for 30 minutes. Then, 10 mL of a 20 % piperidine solution was added, and a first deprotection was performed at 70 °C for 10 minutes. Thereafter, a 1 mL aliquot of the supernatant was collected and placed in an Ep tube, and a coupling reaction was performed while varying the coupling reaction time (30 seconds to 240 seconds). And after washing, a second deprotection was performed, and a 1 mL aliquot of the supernatant was collected and placed in an Ep tube. The supernatants from the first and second deprotections were diluted by the same factor, and UV spectra were measured.

**[Table 3]**

| Step | Reagent (Solvent: DMF) | Volume (mL) | Temperature (°C) | Time (min) |
|---|---|---|---|---|
| Deprotection 1 | 20 % piperidine | 10 | 70 | 10 |
| Washing | DMF | 10 (5 times) | - | - |
| Coupling | Fmoc-Amino acid (0.2 M) | 6 | 70 | * |
| Coupling | DIC (0.5 M) | 2 | 70 | * |
| Coupling | Oxyma pure (1.0 M) | 1 | 70 | * |
| Washing | DMF | 10 (5 times) | - | - |
| Deprotection 2 | 20 % piperidine | 10 | 70 | 10 |

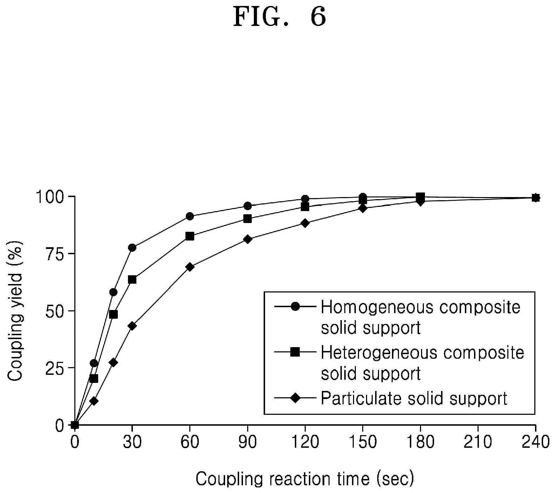

Setting the absorbance at 301 nm of the supernatant from the first deprotection to 100 %, the coupling reaction yield (Coupling Yield (%)) was calculated using the absorbance of the supernatant from the second deprotection. As shown in FIG. 6, the homogeneous and heterogeneous composite solid supports according to the Examples exhibited high coupling reaction yields even at short coupling reaction times compared to the conventional particulate solid support. In particular, the homogeneous composite solid support using the active monomer participating together in the curing reaction exhibited a superior coupling reaction yield even compared to the heterogeneous composite solid support, confirming that the homogeneous composite solid support is most suitable for use as a solid support for synthesizing biological polymers.

### Example 7. Synthesis Test Using Batch-Type Automated Reactor

Synthesis of an ACP (acyl carrier protein) model peptide (ACP 65-74) was performed using a CEM Liberty Blue automated reactor.
SEQ ID NO: 1: VQAAIDYING

0.1 mmol each of the particulate solid support and the homogeneous composite solid support of Example 3 were separately added to respective batch-type reactors, and the reaction steps in Table 4 below were repeatedly performed under the same conditions while changing only the type of amino acid.

**[Table 4]**

| Step | Reagent (Solvent: DMF) | Volume (mL) | Temperature (°C) | Time (min) |
|---|---|---|---|---|
| Deprotection | 20 % piperidine, 0.1 M Oxyma pure | 15 | 70 | 2 |
| Washing | DMF | 10 (3 times) | - | - |
| Coupling | Fmoc-Amino acid (0.2 M) | 6 | 70 | 4 |
| Coupling | DIC (0.5 M) | 2 | 70 | 4 |
| Coupling | Oxyma pure (1.0 M) | 1 | 70 | 4 |
| Washing | DMF | 10 (2 times) | - | - |

After the reactions were completed, the particulate solid support and the homogeneous composite solid support were each sufficiently washed with EtOH and dried, and then a cleavage reaction was performed for 2 hours. The cleavage reaction solution used at this time was a mixture of 95 % TFA (trifluoroacetic acid), 2.5 % TIS (triisopropylsilane), and 2.5 % DW (distilled water). After cleavage, washing was performed by precipitating the cleaved peptides using cold ether and then centrifuging the precipitates. The ether washing was performed twice, and after the precipitates were sufficiently dried by a reduced-pressure drying method, the purity of the synthesized peptide was measured using high-performance liquid chromatography (HPLC) (Table 5).

**[Table 5]**

| Classification | Purity (%) | Yield (%) |
|---|---|---|
| Homogeneous composite solid support | 85.1 | 88.4 |
| Particulate solid support | 81.4 | 71.5 |

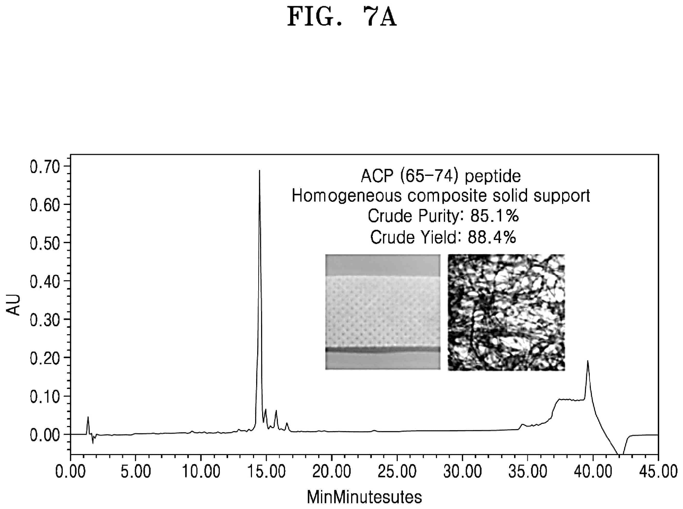

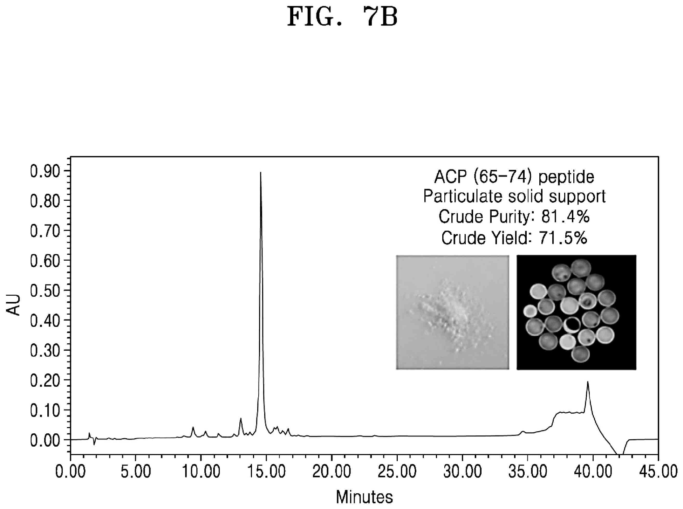

FIGS. 7A and 7B are graphs confirming the purities and yields of ACP (65-74) synthesized using the homogeneous composite solid support of the Example and the conventional particulate solid support. As shown in Table 5 and FIGS. 7A and 7B, as a result of the synthesis test analysis using a conventional commercial SPPS batch-type reactor (Liberty Blue, CEM), the purity of the ACP (65-74) peptide synthesis was found to be slightly improved when using the homogeneous composite solid support of the Example, but the yield was found to be significantly superior. These values indicate that both the conventional particulate solid support and the homogeneous composite solid support exhibit levels suitable for industrial use in a batch-type automated reactor, and in particular, the homogeneous composite solid support exhibits significantly superior yield, indicating that the homogeneous composite solid support is more suitable for a batch-type reactor.

### Example 8. Synthesis Test Using Flow-Type Reactor

To compare the synthesis performances of the particulate solid support and the homogeneous composite solid support in a flow-type reactor, 0.5 mmol each of the particulate solid support and the homogeneous composite solid support of Example 3 were separately added to respective 27 mL columns, and the reaction steps were performed under the conditions shown in Table 6 below while changing the type of amino acid to synthesize the ACP (acyl carrier protein) model peptide (65-74) of SEQ ID NO: 1. Thereafter, the yield was calculated by measuring the dry mass of the synthesized peptide, and the purity of the synthesized peptide was measured using high-performance liquid chromatography (HPLC).

**[Table 6]**

| Reaction step | Reagent (Solvent: DMF) | Volume (mL) | Temperature (°C) | Time (min) | Flow rate (mL/min) |
|---|---|---|---|---|---|
| Deprotection | 20 % piperidine, 0.1 M Oxyma pure | 25 | 70 | 2 | 100 |
| Washing | DMF | 125 | - | - | |
| Coupling | Fmoc-Amino acid (0.2 M) | 15 | 70 | 4 | |
| | DIC (0.5 M) | 6 | | | |
| | Oxyma pure (1.0 M) | 3 | | | |
| Washing | DMF | 20 | - | - | |

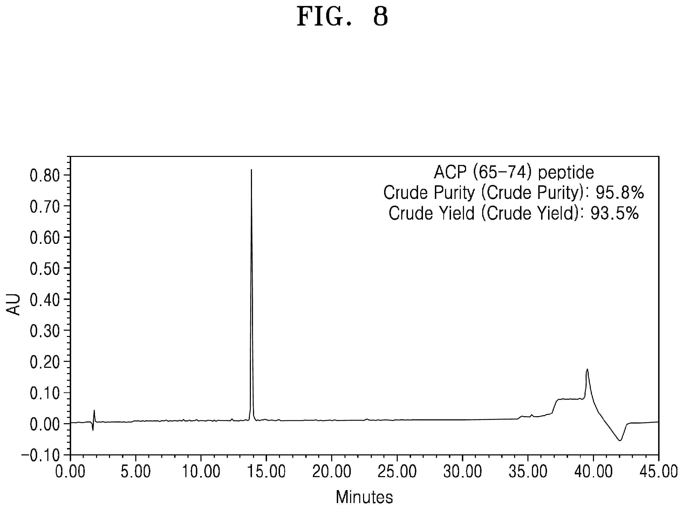

As shown in FIG. 8, the synthesis performance in the flow-type reactor using the homogeneous composite solid support of the Example was found to be superior in all aspects of synthesis purity and yield compared to the batch-type reactor of Example 7. On the other hand, when the ACP (65-74) peptide synthesis was performed by packing the flow-type reactor with the particulate solid support, a backpressure issue in which the column became clogged occurred during the synthesis, and thus the synthesis was not properly performed. This is caused by the particulate solid support clogging the column of the flow-type reactor due to swelling, indicating that the composite solid support, which exists in a state in which the functional coating is coated on the structure core, does not give rise to such an issue, and thus is more suitable for use in a flow-type reactor.

### Example 9. Peptide Synthesis Test According to Type of First Monomer of Homogeneous Composite Solid Support

To compare the loading density and peptide synthesis performance of the homogeneous composite solid supports prepared according to the length of the first monomer, homogeneous composite solid supports having different lengths of the first monomer were prepared, and an experiment was performed. Specifically, the same reagents and conditions as those in Table 1 of Example 1 were used to prepare the homogeneous composite solid supports. At this time, as the first monomer, three types of polyethylene glycol diacrylate (PEG) having different n values in C₃H₃O(OCH₂CH₂)ₙC₃H₃O₂ (n=4, n=9, and n=14) were used.

### Example 9-1: Measurement of Loading Density of Homogeneous Composite Solid Support

8 mL of DMF was added to each homogeneous composite solid support, and the solid supports were allowed to swell for 15 minutes. 2 mL of a 20 % piperidine solution was added to the sufficiently swollen homogeneous composite solid supports, and after the mixture was reacted at room temperature for 45 minutes, a 1 mL aliquot of the supernatant was collected. After the aliquot was diluted until the absorbance of the supernatant reached approximately 1, the absorbance was measured, and the interval between reaction sites was calculated using Equation 1.

### Example 9-2: Synthesis of Peptide Using Prepared Solid Support for Synthesizing Biological Polymers

1.13 g, 1.09 g, and 1.21 g of the homogeneous composite solid supports using the three types of PEGs having different lengths (PEG-4, PEG-9, and PEG-14), respectively, were placed into respective 27 mL synthesis columns, washed with 25 mL of DMF, and then circulated at 70 °C at a rate of 100 mL/min for 10 minutes. 1.63 g of Fmoc-Rink-Linker was dissolved in 15 mL of DMF in a conical tube, and then 6 mL of 0.5 M DIC and 3 mL of 1.0 M Oxyma pure were added. The solution in the conical tube was placed into the synthesis columns and circulated at 70 °C for 10 minutes. Thereafter, after the synthesis membranes were washed with 50 mL of DMF, 25 mL of a deprotection solution (0.1 M Oxyma pure, 20 % piperidine in DMF) was circulated in the synthesis columns at 50 °C for 4 minutes, and then the synthesis membranes were washed with 125 mL of DMF.

0.95 g of Fmoc-Gly-OH was dissolved in 15 mL of DMF in another conical tube, and then 6 mL of 0.5 M DIC and 3 mL of 1.0 M Oxyma pure were added. After stirring for 5 minutes, the solution was placed into the columns and circulated at 70 °C for 10 minutes. Thereafter, the synthesis membranes were washed with 50 mL of DMF.

The following compounds were sequentially added to proceed with a chain reaction for peptide synthesis: 1.91 g of Fmoc-Asn(Trt)-OH, 1.13 g of Fmoc-Ile-OH, 1.47 g of Fmoc-Tyr(tBu)-OH, 1.32 of Fmoc-Asp(OtBu)-OH, 1.13 g of Fmoc-Ile-OH, 0.99 g of Fmoc-Ala-OH, 0.99 g of Fmoc-Ala-OH, 1.95 g of Fmoc-Gln(Trt)-OH, and 1.09 g of Fmoc-Val-OH.

25 mL of a deprotection solution (0.1 M Oxyma pure, 20 % piperidine in DMF) was added to the reaction columns and circulated at 50 °C for 4 minutes, and then the solid supports were washed with 125 mL of DMF and 125 mL of ethanol. Thereafter, drying under reduced pressure was performed for 1 hour to obtain the homogeneous composite solid supports on which the dried peptides were synthesized.

To 3 conical tubes, a cleavage solution (TFA:TIS:D.W. = 95:2.5:2.5) was added in an amount of 10 mL each, and the homogeneous composite solid supports on which the peptides were synthesized were placed into the respective tubes and the mixtures were reacted at room temperature for 2 hours. Thereafter, the solutions in the tubes were collected, and then precipitation was performed by adding 20 mL of cold diethyl ether to each. The precipitated solutions were centrifuged to obtain solids, and after the solids were washed two more times using 20 mL of cold ether each, the solids were sufficiently dried using a reduced-pressure drying method to obtain the peptide of SEQ ID NO: 1.

The dry masses of ACP (65-74) synthesized using the three types of synthesis membranes were measured to calculate the crude yields, and high-performance liquid chromatography (HPLC) and MALDI-TOF/TOF were performed to measure the purities, and the results are shown in Table 7.

**[Table 7]**

| Type of PEG | Loading density of functional coating (mmol/g) | Purity (%) | Yield (%) |
|---|---|---|---|
| n=4 | 0.25 | 85.2 | 84.4 |
| n=9 | 0.16 | 98.2 | 94.3 |
| n=14 | 0.12 | 99.4 | 95.6 |

Through Table 7, it could be confirmed that the greater the interval between the reaction sites of the solid support for synthesizing biological polymers due to the PEG, the higher the yield and purity with which the synthesized peptide was synthesized.

From the above results, it was confirmed that as the length of the first monomer PEG increases, the loading density of the functional coating decreases, but the purity and yield of the peptide synthesis increase. That is, when the length of the first monomer is long, the distance between the reaction sites in the functional coating may increase, and thus the purity and yield may increase. However, when the length of the first monomer is excessively long, the loading density conversely decreases, which may render the composite solid support unsuitable for applications requiring synthesis of a large amount of peptide. In addition, when the length of the first monomer is short, the loading density may increase, but when the length is excessively short, the swelling characteristic becomes excessively large, which may cause a problem of reduced durability. Therefore, an appropriate selection of the length of the first monomer is required depending on the target to be synthesized, and conversely, since the composite solid support of the Example can adjust the loading density and purity/yield through the adjustment of the length of the first monomer, it can be interpreted that the composite solid support can be used for synthesizing various biological polymers.

### Example 10. Synthesis Test by Flow Rate in Flow-Type Reactor

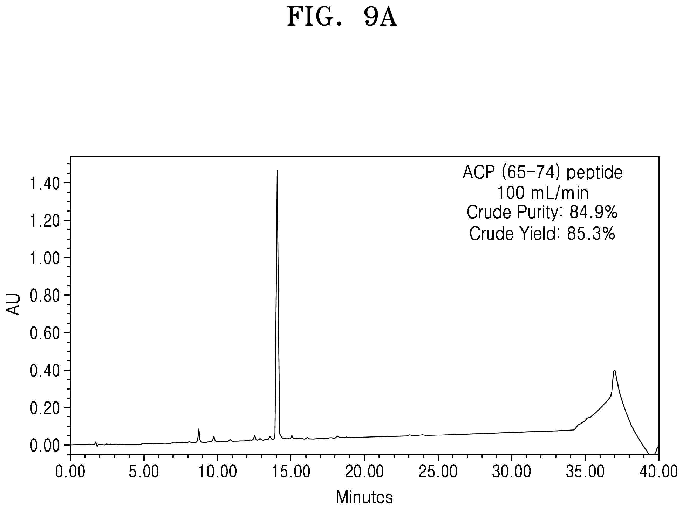

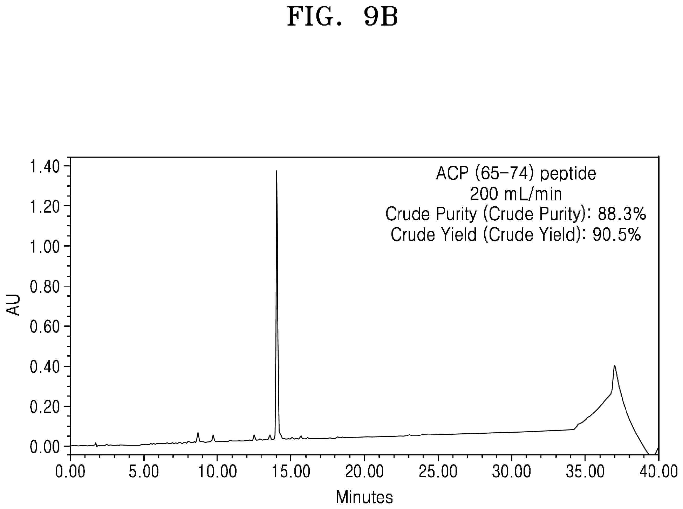

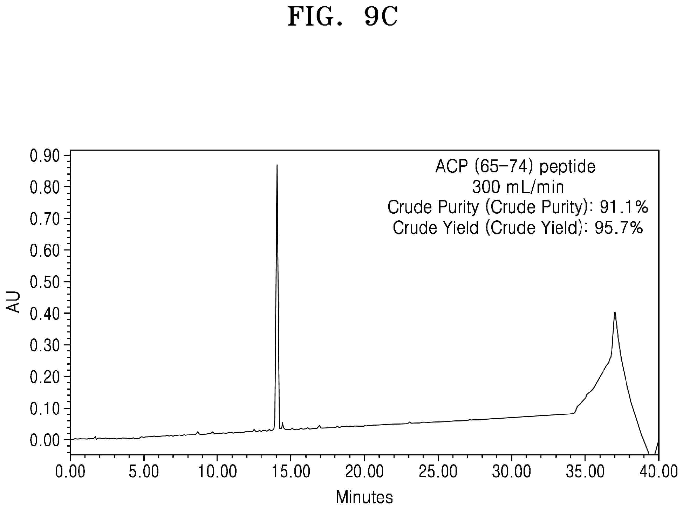

Using the composite solid support of Example 3, the peptide synthesis yield and purity according to the flow rate in the flow-type reactor were confirmed. ACP (65-74) was synthesized in the same manner as in Example 9, and the results of the crude purity and yield of the synthesized ACP (65-74) peptide are as shown in Table 8 and FIGS. 9A to 9C.

**[Table 8]**

| Flow rate (mL/min) | Purity (%) | Yield (%) |
|---|---|---|
| 100 | 84.9 | 85.3 |
| 200 | 88.3 | 90.5 |
| 300 | 91.1 | 95.7 |

FIGS. 9A to 9C show HPLC graphs for ACP (65-74) synthesized according to the flow rate in the flow-type reactor using the composite solid support of the Example.

As shown in the drawings and Table 8, it was confirmed that as the flow rate of the column increased, the purity and yield of the peptide increased. Through this, it can be seen that the composite solid support of the Example exhibits a purity and yield of 80 % or more even at various flow rates, and thus the composite solid support can be suitably used in a flow-type reactor that needs to synthesize various biological polymers.

### Example 11. Comparison of Peptide Synthesis Purity According to Surface Treatment of Structure Core in Preparation of Homogeneous Composite Solid Support and Heterogeneous Composite Solid Support

As in Example 4, the synthesis purities of the peptide of SEQ ID NO: 2 were compared according to the cases of using a solid support including an untreated structure core and a solid support including a surface-treated structure core.
SEQ ID NO: 2: WFTTLISTIM

First, prior to impregnating the structure core in the functional coating solution in Example 3, various types of surface hydrophilic treatments were performed. The types of hydrophilic treatments are as shown in Table 9.

**[Table 9]**

| Surface hydrophilic treatment method | | |
|---|---|---|
| Chemical method | Surfactant | Impregnation in an aqueous surfactant solution such as sodium dodecyl sulfate (SDS), sodium dodecylbenzenesulfonate (SDBS), or Triton X-100, followed by drying |
| | Acidic solution | Impregnation in an aqueous acid solution such as hydrochloric acid, acetic acid, acetic anhydride, or phosphoric acid for a predetermined time, followed by washing with a large amount of water and drying |
| Physical method | Plasma | Performing a surface treatment using a plasma generator |
| | UV | Performing a surface treatment using a UV light source |

Each prepared composite solid support was introduced into a column, washed with 25 mL of DMF, and then circulated with 25 mL of DMF for 10 minutes. Thereafter, 6 equivalents of Fmoc-Rink amide MBHA Linker-OH, DIC, and Oxyma pure were dissolved in 25 mL of DMF, and the solution was circulated at 70 °C for 10 minutes at a flow rate of 100 mL. Then, the composite solid support was washed twice with 25 mL of DMF, and 25 mL of a deprotection solution (0.1 M Oxyma pure, 20 % piperidine in DMF) was circulated at 50 °C for 4 minutes. Finally, the composite solid support on which the functional coating was formed was washed twice with 50 mL of DMF and once with 25 mL of DMF. Thereafter, Fmoc-Rink amide MBHA Linker-OH was sequentially replaced with the following compounds, and the procedure from the initial washing using DMF to the washing of the composite solid support on which the functional coating was formed with DMF was repeatedly performed: [Fmoc-Met-OH, Fmoc-Ile-OH, Fmoc-Thr(tBu)-OH, Fmoc-Ser(tBu)-OH, Fmoc-Ile-OH, Fmoc-Leu-OH, Fmoc-Thr(tBu)-OH, Fmoc-Thr(tBu)-OH, Fmoc-Phe-OH, Fmoc-Trp(Boc)-OH]

Finally, the composite solid support on which the peptide was formed was obtained, and the composite solid support was washed three times with 25 mL of ethanol. After drying, a cleavage reaction was performed on the composite solid support for 2 hours. The cleavage reaction solution used here was a mixture of 95 % TFA, 2.5 % TIS, and 2.5 % DW. After the cleavage reaction, ether washing was performed by precipitating the cleaved peptides using cold ether and centrifuging the precipitates. The ether washing was performed twice, and thereafter, the precipitates were sufficiently dried by a reduced-pressure drying method.

**[Table 10]**

| Hydrophilic treatment | Purity (%) |
|---|---|
| Untreated | 58.16 |
| SDS | 62.41 |
| Triton X-100 | 65.61 |
| Acetic acid | 67.86 |
| Hydrochloric acid | 65.17 |
| Plasma | 65.59 |
| UV | 62.26 |

Through Table 10 above, the purities of the peptide syntheses using the composite solid supports prepared differently according to the hydrophilic treatment method of the structure core can be confirmed. First, it was confirmed that the composite solid support using the untreated structure core resulted in lower purity compared to the composite solid support using the hydrophilically treated structure core. In addition, regarding the hydrophilic treatment method, no significant difference was observed between the chemical method and the physical method. Therefore, in synthesizing a biological polymer using the composite solid support of the Example, it was confirmed that performing a hydrophilic treatment on the structure core during the preparation process is more advantageous for the synthesis, and the hydrophilic treatment used here can be applied regardless of whether it is a physical or chemical method.

### Example 12. Preparation of Composite Solid Support According to Functional Coating Content and Peptide Synthesis Test

### Example 12-1: Adjustment of Functional Coating Content

A composite solid support was prepared in the same manner as in Example 3, except that the amount of the functional coating coated on the structure core was adjusted. Specifically, the structure core was sufficiently impregnated with the functional coating solution, and after the structure core was removed from the coating solution, the structure core was passed between rollers having a constant gap to remove a certain amount of the functional coating solution absorbed by the solid support. The adjustment of the content of the functional coating coated on the solid support was performed according to the masses and thicknesses in Table 11 below.

### Example 12-2: Measurement of Porosity and Synthesis of Peptide

A solid support without a functional coating (polypropylene, spunbond non-woven fabric, density 0.89 g/cm³) was cut to 10 cm × 10 cm, its mass and thickness were measured, and then the porosity was calculated (CTR 0). Here, the thickness was determined by measuring the specimen at 10 points at 0.5 cm intervals and averaging the measured values, wherein a thickness under a constant pressure applied using a ratchet stop was used. In addition, the masses and thicknesses of the previously prepared composite solid supports were measured in the same manner, and then the porosity reduction was calculated (CTR 1 to 6).

**[Table 11]**

| | CTR 0 | CTR 1 | CTR 2 | CTR 3 | CTR 4 | CTR 5 | CTR 6 |
|---|---|---|---|---|---|---|---|
| Mass (mg) | 295 | 315 | 368 | 420 | 537 | 652 | 801 |
| Thickness (mm) | 0.258 | 0.255 | 0.261 | 0.259 | 0.262 | 0.264 | 0.272 |
| Area (mm²) | 10000 | 10000 | 10000 | 10000 | 10000 | 10000 | 10000 |
| Density (g/cm³) | 0.89 | 0.89 | 0.89 | 0.89 | 0.89 | 0.89 | 0.89 |
| Porosity (%) | 87 | 86 | 84 | 82 | 77 | 72 | 67 |
| ΔPorosity | 0.00 | -1.03 | -2.99 | -5.37 | -10.18 | -14.90 | -20.24 |

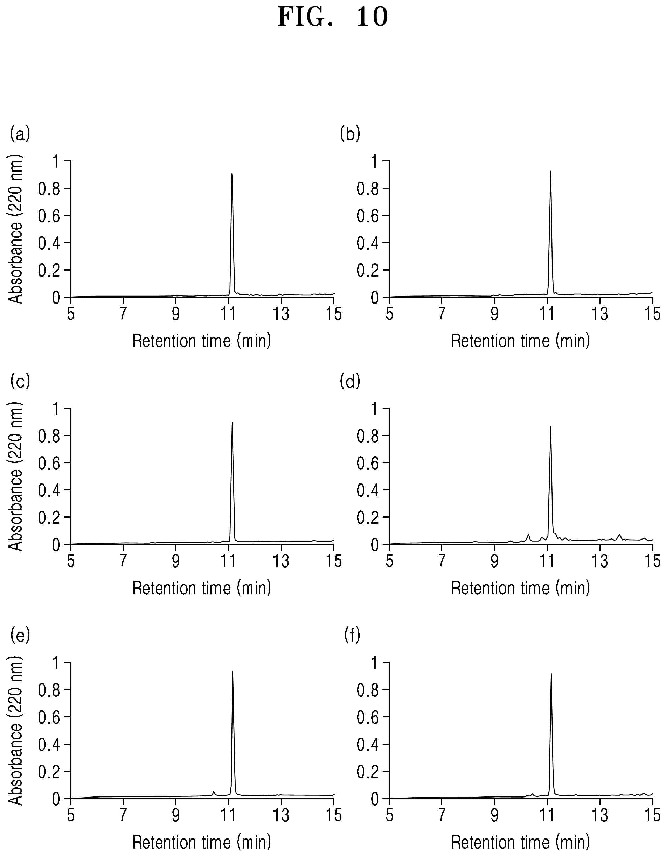

To test the peptide synthesis of the composite solid support according to Δporosity, the ACP 65-74 peptide was synthesized in the same manner as in Example 8, and its purity was confirmed by HPLC. The synthesis results are as shown in Table 12 and FIG. 10, and it was confirmed that as the thickness and mass of the applied functional coating increased, the porosity decreased, but the peptide synthesis purity gradually increased. In particular, the highest synthesis purity was observed at CTR 2, and the desired level of synthesis purity was observed up to CTR 5. However, when the coating thickness became excessively thick, the synthesis purity instead decreased, which is interpreted as a result of the reaction solution failing to flow smoothly because the porosity became excessively low.

**[Table 12]**

| | CTR 0 | CTR 1 | CTR 2 | CTR 3 | CTR 4 | CTR 5 | CTR 6 |
|---|---|---|---|---|---|---|---|
| ΔPorosity | 0.00 | -1.03 | -2.99 | -5.37 | -10.18 | -14.90 | -20.24 |
| Purity (%) | 0.00 | 95.5 | 96.2 | 95.8 | 93.7 | 87.2 | 57.6 |

### Example 13. Synthesis of Various Biological Polymers Using Composite Solid Support

To confirm whether the solid-phase support synthesis technology using the composite solid support is not limited to peptide synthesis, but can synthesize various types of sequence-defined biopolymers such as peptide nucleic acids (PNAs), peptoids, and oligourea foldamers, the following experiment was performed.

### Example 13-1: Synthesis of PNA-Peptide Using Flow-Type Reactor

First, 1.5 g of the composite solid support was placed into a reaction column and washed with 25 mL of DMF, and then DMF was circulated at 70 °C at a rate of 100 mL/min for 10 minutes. 1.62 g of Fmoc-Rink-Linker was dissolved in 15 mL of DMF in a conical tube, and then 3 mL of 1 M DIC and 6 mL of 0.5 M Oxyma pure were added. After the solution in the tube was transferred to the column, the solution was circulated at 70 °C for 10 minutes, and then the synthesis membrane was washed with 50 mL of DMF.

Thereafter, 25 mL of a deprotection solution (0.1 M Oxyma pure, 20 % piperidine in DMF) was circulated in the column at 50 °C for 4 minutes, and then the synthesis membrane was washed with 125 mL of DMF. 178 mg of Fmoc-Gly-OH was dissolved in 15 mL of DMF in a conical tube, and then 3 mL of 1.0 M DIC and 6 mL of 0.5 M Oxyma pure were added. After stirring for 5 minutes, the solution was transferred to the column, the solution was circulated at 70 °C for 10 minutes, and then the synthesis membrane was washed with 50 mL of DMF.

Thereafter, Fmoc-Gly-OH was sequentially replaced with the following compounds to proceed with a chain reaction for PNA-peptide synthesis: [445 mg of Fmoc-G(Bhoc)-OH, 304 mg of Fmoc-T-aeg-OH, 421 mg of Fmoc-C(Bhoc)-OH, and 435 mg of Fmoc-A(Bhoc)-aeg-OH]. Thereafter, 25 mL of the deprotection solution was added to the column, and the solution was circulated at 50 °C for 4 minutes, and then the synthesis membrane was washed with 125 mL of a mixed solvent and 125 mL of ethanol. Drying under reduced pressure was performed for 1 hour to prepare the PNA-peptide of SEQ ID NO: 3.
SEQ ID NO: 3: ACTG-Gly

To a conical tube, a cleavage solution (TFA:thioanisole:D.W. :PhOH:EDT = 82.5:5:5:5:2.5) was added in an amount of 15 mL, and after the NH₂-ACTG-Gly-composite solid support was placed therein, the mixture was reacted at room temperature for 3 hours. Only the solution in the tube was collected, and the solution was added to 120 mL of cold diethyl ether to proceed with precipitation. The precipitated solution was centrifuged to obtain a solid, and after 120 mL of cold ether was added to wash the solid two more times, the solid was sufficiently dried using a reduced-pressure drying method.

### Example 13-2: Synthesis of PNA-Peptide Using Conventional Solid-Phase Synthesis Method

First, 0.25 g of Fmoc-Rink amide MBHA resin as the particulate solid support was added to a reactor, and then 5 mL of DMF was added, and the mixture was stirred for 10 minutes. Thereafter, 5 mL of a deprotection solution was added, and the mixture was mixed using nitrogen at room temperature for 5 minutes. This was repeated one more time. The reactor was washed three times using 10 mL of a mixed solution (DMF:DCM = 1:1). 1.5 mL of 0.2 M Fmoc-Gly-OH, 0.6 mL of 0.5 M DIC, 0.3 mL of 1.0 M Oxyma pure, 3 mL of 1.0 M DIPEA, and 0.3 mL of 1.0 M 2,6-lutidine were added to a conical tube. After the solutions in the tube were mixed, the solution was transferred to the reactor, and the mixture was reacted at room temperature for 30 minutes. This was repeated one more time. Next, the reactor was washed six times using 10 mL of the mixed solution.

Thereafter, Fmoc-Gly-OH was sequentially replaced with the following compounds to proceed with a chain reaction for PNA-peptide synthesis: [Fmoc-G(Bhoc)-OH, Fmoc-T-aeg-OH, Fmoc-C(Bhoc)-OH, Fmoc-A(Bhoc)-aeg-OH]. Thereafter, 5 mL of the deprotection solution was added, and the mixture was mixed using nitrogen at room temperature for 5 minutes. This was repeated one more time. The reactor was washed three times using 10 mL of mixed solution 2 to prepare the PNA of SEQ ID NO: 3.

Thereafter, to a conical tube, a cleavage solution (TFA:TIS:D.W. :PhOH:EDT = 82.5:5:5:5:2.5) was added in an amount of 5 mL. The synthesized NH₂-ACTG-Gly-composite solid support was placed therein, and the mixture was reacted at room temperature for 3 hours. Only the solution in the tube was collected, and the TFA was removed using nitrogen. The collected solution was introduced into 15 mL of cold ether to proceed with precipitation. The precipitated solution was centrifuged to obtain a solid, and after the solid was washed two more times using 15 mL of cold ether, the solid was sufficiently dried using a reduced-pressure drying method.

The dry masses of the PNA-peptides synthesized using the composite solid support and the conventional particulate solid support were measured to calculate the crude yields, and high-performance liquid chromatography (HPLC) and MALDI-TOF/TOF were performed to measure the purities, and the results are shown in Table 13.

**[Table 13]**

| Solid support | Purity (%) | Yield (%, with TFA salt) |
|---|---|---|
| Homogeneous composite solid support | 90.0 | 105.3 |
| Particulate solid support | 86.8 | 103.0 |

As shown in Table 13, the yield and purity of the PNA-peptide synthesized using the composite solid support of the Example were improved compared to the PNA-peptide synthesized using the conventional particulate solid support. From this, it was confirmed that the composite solid support can be used for synthesizing various types of biological polymers such as peptide nucleic acids, in addition to peptides such as ACP (65-74).

The foregoing description of the present disclosure is for illustrative purposes, and those of ordinary skill in the art to which the present disclosure pertains will be able to understand that the present disclosure can be readily modified into other specific forms without changing the technical spirit or essential features of the present disclosure. Therefore, it should be understood that the embodiments described above are illustrative in all respects and not restrictive. For example, each component described as a single type may be implemented in a distributed manner, and similarly, components described as being distributed may also be implemented in a combined form.

The scope of the present disclosure is defined by the appended claims rather than the foregoing detailed description, and all changes or modifications derived from the meaning and scope of the claims and equivalent concepts thereof should be construed as being included in the scope of the present disclosure.

## Claims

1. A composite solid support for synthesizing a biological polymer, the composite solid support comprising:
a structure core having a length of 1 mm or more and a shape of at least one dimension; and
a functional coating disposed on the structure core,
wherein the biological polymer is synthesized in the functional coating.

2. The composite solid support for synthesizing a biological polymer of claim 1,
wherein the functional coating has a characteristic of swelling in a solvent.

3. The composite solid support for synthesizing a biological polymer of claim 1,
wherein the functional coating comprises a functional group on a surface thereof, in an interior thereof, or both.

4. The composite solid support for synthesizing a biological polymer of claim 1,
wherein a loading density of the functional coating is 0.01 mmol/g to 2 mmol/g.

5. The composite solid support for synthesizing a biological polymer of claim 1,
wherein the functional coating occupies 1 % to 15 % of a pore volume of the structure core without the functional coating.

6. The composite solid support for synthesizing a biological polymer of claim 1,
wherein a mass of the functional coating is 5 % to 200 % relative to a mass of the structure core without the functional coating.

7. The composite solid support for synthesizing a biological polymer of claim 1,
wherein the structure core has a characteristic of solvent resistance, thermal resistance, or both.

8. The composite solid support for synthesizing a biological polymer of claim 1,
wherein a component of the structure core comprises one or more selected from a polymer, a metal, and a ceramic.

9. The composite solid support for synthesizing a biological polymer of claim 1,
wherein the structure core has one or more shapes selected from a one-dimensional shape, a two-dimensional shape, and a three-dimensional shape.

10. The composite solid support for synthesizing a biological polymer of claim 9,
wherein the one-dimensional shape comprises one or more selected from a staple fiber, a filament fiber, and a rod.

11. The composite solid support for synthesizing a biological polymer of claim 9,
wherein the two-dimensional shape comprises one or more selected from a spunbond non-woven fabric, a meltblown non-woven fabric, a needle-punched non-woven fabric, a hydroentangled non-woven fabric, a woven fabric, a knitted fabric, a porous membrane, a polymeric film, and a mesh.

12. The composite solid support for synthesizing a biological polymer of claim 9,
wherein the three-dimensional shape comprises an open-cell foam, a macro-pored sphere, or both.

13. The composite solid support for synthesizing a biological polymer of claim 1,
wherein the functional coating comprises a polymer of one or more main monomers and an active monomer.

14. The composite solid support for synthesizing a biological polymer of claim 2,
wherein the functional coating has an expansion amount per unit mass of 2 mL/g to 8 mL/g in water.

15. A method of preparing a composite solid support for synthesizing a biological polymer, the method comprising:
preparing a functional coating solution by mixing one or more main monomers, an active monomer, an initiator, and a solvent;
impregnating a structure core having a length of 1 mm or more and a shape of at least one dimension with the functional coating solution; and
polymerizing the functional coating solution applied to the structure core.

16. The method of preparing a composite solid support for synthesizing a biological polymer of claim 15,
wherein the structure core impregnated with the functional coating solution has a surface that is hydrophilically treated.

17. The method of preparing a composite solid support for synthesizing a biological polymer of claim 15,
wherein a loading density is adjusted according to a length of the main monomer.

18. A method of synthesizing a biological polymer, the method comprising:
coupling a first amino acid residue to which a protecting group is linked to the composite solid support of claim 1;
removing the protecting group; and
coupling a second amino acid residue to which a protecting group is linked to an N-terminus or a C-terminus of the previously coupled first amino acid from which the protecting group has been removed.
